# EUROPEAN PATENT APPLICATION

(11) **EP 4 692 034 A1**
(43) Date of publication of application: **11.02.2026**
(21) Application number: 24850341.9
(22) Date of filing: 24.07.2024
(51) Int. Cl.: C07C 29/141, C07C 29/80, C07C 31/20, C07C 45/75, C07C 45/82, C07C 47/19

(54) **METHOD FOR PREPARING NEOPENTYL GLYCOL**

(30) Priority: 20.09.2023 KR 20230125459
(71) Applicant: LG Chem, Ltd., Seoul 07336 (KR)
(72) Inventor: KIM, Sung Kyun, Daejeon 34122 (KR); LEE, Sung Kyu, Daejeon 34122 (KR); CHO, Seung Sik, Daejeon 34122 (KR); LEE, Min Seok, Daejeon 34122 (KR)
(74) Representative: Goddar, Heinz J.
(86) International application number: PCT/KR2024/010684
(87) International publication number: WO 2025/063472

(57) **Abstract**

The present invention provides a method for preparing neopentyl glycol, the method including: feeding a feed stream containing formaldehyde and isobutyraldehyde to an aldol reactor and subjecting the feed stream to an aldol condensation reaction in the presence of a catalyst to obtain a first reaction product containing hydroxypivaldehyde; feeding the first reaction product to an aldol purification column; feeding the aldol purification column upper discharge stream to a decanter and separating an organic phase stream containing unreacted isobutyraldehyde and the catalyst and an aqueous phase stream containing a catalyst salt; feeding the aqueous phase stream to a saponification reactor to reduce the catalyst salt to a catalyst and recover the catalyst; and feeding the aldol purification column lower discharge stream to a hydrogenation reactor and subjecting the aldol purification column lower discharge stream to a hydrogenation reaction to obtain neopentyl glycol.

## Description

### [Technical Field]

### CROSS-REFERENCE TO RELATED APPLICATION

The present application claims the benefit of priority to Korean Patent Application No. 10-2023-0125459, filed on September 20, 2023, the entire contents of which are incorporated herein by reference.

### Technical Field

The present invention relates to a method for preparing neopentyl glycol, and more particularly, to a method for preparing high-purity neopentyl glycol without using an extractant.

### [Background Art]

Neopentyl glycol may be generally prepared by subjecting isobutyraldehyde and formaldehyde to an aldol condensation reaction in the presence of a catalyst to form hydroxypivaldehyde, and then performing a hydrogenation reaction on the hydroxypivaldehyde.

In this case, in order to subject the hydroxypivaldehyde to the hydrogenation reaction, a process of separating hydroxypivaldehyde from a reaction product obtained after the aldol condensation reaction is essential. In the related art, in order to separate hydroxypivaldehyde from the reaction product, an extraction process was performed to extract the hydroxypivaldehyde using an extractant, for example, 2-ethylhexanol. However, in the case of using an extractant, there was a problem that the process design was complicated and facility costs and energy usage increased because a separate distillation device was required to use and recover the extractant. In addition, as the extractant was used, it may have been difficult to obtain high-purity neopentyl glycol because the extractant may have remained in neopentyl glycol, which is a final product. Therefore, there is a need for a process capable of obtaining high-purity neopentyl glycol without using an extractant.

Meanwhile, formic acid is produced from the Cannizzaro side reaction that occurs in the aldol condensation reaction, and the catalyst is converted to a catalyst salt by the formic acid. The catalyst salt has been treated as wastewater in the form of an aqueous phase in the related art. In addition, hydroxypivalic acid-neopentylglycol ester (HPNE) that is produced through the Tishchenko reaction, which is another side reaction of the aldol condensation reaction process, has been discarded entirely together with high boiling point by-products during a process of purifying neopentyl glycol. However, the hydroxypivalic acid-neopentylglycol ester (HPNE) needs to be recovered and utilized because it is a high-value-added product in itself.

That is, there is an environmental pollution problem caused by the discarded catalyst salt and HPNE, and there is a problem that the production costs increase because a new catalyst has to be continuously added as much as it is discarded due to being produced as a catalyst salt.

Therefore, there is a need to introduce an environmentally friendly process that may reduce cost of energy by recovering the discarded catalyst and HPNE.

### [Disclosure]

### [Technical Problem]

In order to solve the problems mentioned in the background art, an object of the present invention is to obtain high-purity neopentyl glycol without using an extractant and to provide an effect that is environmentally friendly and may further reduce cost of energy in the overall process.

### [Technical Solution]

In one general aspect, a method for preparing neopentyl glycol includes feeding a feed stream containing formaldehyde and isobutyraldehyde to an aldol reactor and subjecting the feed stream to an aldol condensation reaction in the presence of a catalyst to obtain a first reaction product containing hydroxypivaldehyde; feeding the first reaction product to an aldol purification column and obtaining an aldol purification column upper discharge stream containing unreacted isobutyraldehyde, a catalyst, and a catalyst salt and an aldol purification column lower discharge stream containing hydroxypivaldehyde; feeding the aldol purification column upper discharge stream to a decanter and separating an organic phase stream containing the unreacted isobutyraldehyde and the catalyst and an aqueous phase stream containing the catalyst salt; recovering the unreacted isobutyraldehyde and the catalyst from the organic phase stream, and feeding the aqueous phase stream to a saponification reactor to reduce the catalyst salt to a catalyst and recovering the catalyst; feeding the aldol purification column lower discharge stream to a hydrogenation reactor and subjecting the aldol purification column lower discharge stream to a hydrogenation reaction to obtain a second reaction product containing neopentyl glycol; and obtaining the neopentyl glycol from the second reaction product.

### [Advantageous Effects]

According to the method for preparing neopentyl glycol of the present invention, in separating hydroxypivaldehyde from the first reaction product, unlike the related art in which the extraction process is performed, hydroxypivaldehyde is separated from the first reaction product through the aldol purification column without the extraction process. Through this, since there is no need to additionally provide the respective distillation devices that use and recover the extractant, the process design may be simplified, and accordingly, it is preferable from the viewpoint of economic feasibility such as process facility costs and device costs. In addition, as the extraction process is not performed, an extractant is not used, and there is no possibility of the extractant being contained in the final product. Therefore, neopentyl glycol may be obtained with a high purity.

Furthermore, the content of water contained in the aldol purification column lower discharge stream is controlled by controlling the ratio of the mass flow rate of water contained in the aldol purification column lower discharge stream to the mass flow rate of water contained in the feed stream, such that the total amount of energy used in the process may be reduced, and the conversion rate to neopentyl glycol through the hydrogenation reaction may be further increased.

Meanwhile, the catalyst salt produced after the aldol condensation reaction is reduced to a catalyst through a saponification reaction, and the catalyst is recovered, such that the catalyst of the aldol condensation reaction may be efficiently reused. Through this, neopentyl glycol may be prepared with economic feasibility, and environmental pollution may be reduced.

Furthermore, in the present invention, hydroxypivalic acid-neopentyl glycol ester, which is produced as a by-product of the aldol condensation reaction to obtain neopentyl glycol, may be recovered and obtained as a useful high-value-added product, which is effective in obtaining two types of products through one process. Through this, the production cost for preparing neopentyl glycol may be reduced, thereby improving the economic feasibility of the overall process.

### [Description of Drawings]

FIG. 1 is a process flow diagram illustrating a method for preparing neopentyl glycol according to an embodiment of the present invention.
FIGS. 2 and 3 are process flow diagrams illustrating a method for preparing neopentyl glycol according to comparative examples of the present invention.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

The term "stream" in the present invention may refer to a flow of a fluid in a process, and may also refer to a fluid flowing through a pipe itself. Specifically, the "stream" may refer to both a fluid flowing through a pipe connecting the respective devices to each other itself and a flow of a fluid. In addition, the fluid may refer to one or more of a gas and a liquid.

In the present invention, in devices such as an extraction column, a purification column, a distillation column, and a recovery column, a "lower portion" of the device refers to a point at a height of 90% to 100% from the uppermost portion of the device to a lower position, and specifically, may refer to the lowermost portion (the bottom of the column), unless otherwise specified. Similarly, an "upper portion" of the device refers to a point at a height of 0% to 10% from the uppermost portion of the device to the lower position, and specifically, may refer to the uppermost portion (the top of the column), unless otherwise specified.

In the present invention, in devices such as an extraction column, a purification column, a distillation column, and a recovery column, an operating temperature of the device may refer to a temperature of an upper portion of the device, unless otherwise specified. Similarly, an operating pressure of the device may refer to a pressure of the upper portion of the device, unless otherwise specified.

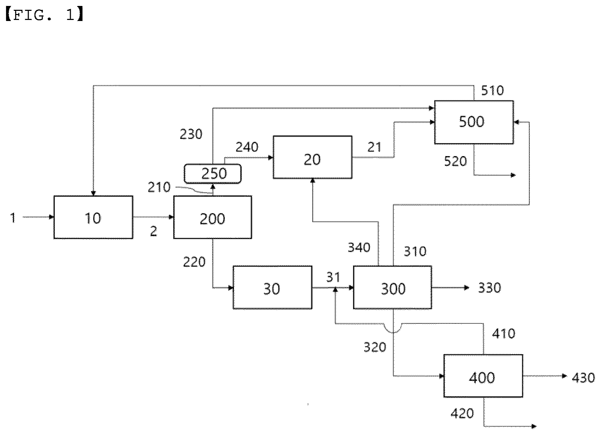

Hereinafter, the present invention will be described in more detail with reference to FIG. 1 in order to assist in the understanding of the present invention.

A method for preparing neopentyl glycol according to an embodiment of the present invention may include subjecting formaldehyde (FA) and isobutyraldehyde (iBAL) to an aldol condensation reaction in an aldol reactor 10 in the presence of a catalyst to obtain a first reaction product containing hydroxypivaldehyde (HPA); feeding the first reaction product to an aldol purification column 200 and obtaining an aldol purification column upper discharge stream containing unreacted isobutyraldehyde, a catalyst, and a catalyst salt and an aldol purification column lower discharge stream containing hydroxypivaldehyde; feeding the aldol purification column upper discharge stream to a decanter 250 and separating an organic phase stream containing the unreacted isobutyraldehyde and the catalyst and an aqueous phase stream containing the catalyst salt; recovering the unreacted isobutyraldehyde and the catalyst from the organic phase stream, and feeding the aqueous phase stream to a saponification reactor 20 to reduce the catalyst salt to a catalyst and recover the catalyst; feeding the aldol purification column lower discharge stream to a hydrogenation reactor 30 and subjecting the aldol purification column lower discharge stream to a hydrogenation reaction to obtain a second reaction product containing neopentyl glycol (NPG); and obtaining the neopentyl glycol from the second reaction product.

First, the method for preparing neopentyl glycol of the present invention may include feeding a feed stream 1 containing formaldehyde and isobutyraldehyde (iBAL) to the aldol reactor 10 and subjecting the feed stream 1 to an aldol condensation reaction in the presence of a catalyst to obtain a first reaction product containing hydroxypivaldehyde (HPA).

The aldol condensation reaction may be performed by reacting formaldehyde and IBAL in the presence of a catalyst in the aldol reactor 10. Specifically, a mixed solution containing the formaldehyde and IBAL may be fed as the feed stream 1 to the aldol reactor 10, and the mixed solution may be subjected to an aldol condensation reaction in the presence of a catalyst in the aldol reactor 10, thereby obtaining a first reaction product containing HPA.

Here, the formaldehyde may be used in an aqueous solution state to secure reactivity and fluidity, and may contain a large amount of water. Such a formaldehyde aqueous solution may contain 30 to 64 wt% of water, and more specifically, 45 to 55 wt% of water, with respect to the total weight of the formaldehyde aqueous solution. In addition, the formaldehyde aqueous solution may further contain methanol to prevent polymerization of formaldehyde. In this case, a content of the methanol may be 0.1 to 15 wt%, and more specifically, 0.1 to 5 wt%, with respect to the total weight of the formaldehyde aqueous solution.

Formalin may be used as the formaldehyde aqueous solution, and in this case, a concentration of the formaldehyde of 35 to 60 wt% may be effective in reducing wastewater.

The catalyst may be an amine-based compound, and specifically, may include a tertiary amine compound such as trialkylamine, trimethylamine, triethylamine, tripropylamine, triisopropylamine, or tributylamine. Preferably, the catalyst may include triethylamine (TEA). In the present invention, the TEA may be used as a catalyst because it has the highest efficiency in the aldol condensation reaction.

An aldol condensation reaction temperature in the aldol reactor 10 may be 70°C to 100°C. When the aldol condensation reaction temperature is lower than 70°C, the aldol condensation reaction may not proceed smoothly due to a low temperature, and thus, it may be difficult to obtain the first reaction product containing HPA at a high conversion rate. When the aldol condensation reaction temperature exceeds 100°C, by-product formation during the aldol condensation reaction may be accelerated.

A residence time in the aldol reactor 10 may be 0.1 hours to 3 hours. When the residence time is shorter than 0.1 hours, an HPA yield may be decreased as the aldol condensation reaction proceeds, and when the residence time is longer than 3 hours, energy efficiency may be reduced and excessive by-products may be produced as the process proceeds for a long time.

In the aldol reactor 10 of the present invention, the aldol condensation reaction may proceed under the conditions described above, and HPA may be produced. In this case, formic acid may be produced by the Cannizzaro side reaction that occurs in the aldol condensation reaction, and the formic acid may react with TEA, which is a catalyst, to produce a TEA salt, that is, a catalyst salt. In addition, hydroxypivalic acid-neopentylglycol ester (HPNE) may be produced by the Tishchenko reaction, which is another side reaction of the aldol condensation reaction. In the related art, it was common to treat and discard the HPNE as a by-product. However, according to an embodiment of the present invention, HPNE may be separated and utilized as a valuable raw material.

As a result, a stream 2 discharged from the aldol reactor 10 may contain HPA, a catalyst salt, and HPNE as the first reaction product. In addition, the first reaction product may further contain unreacted IBAL that is not reacted during the aldol condensation reaction, water, and a catalyst.

Subsequently, the method for preparing neopentyl glycol according to an embodiment of the present invention may include feeding the first reaction product to the aldol purification column 200 and obtaining an aldol purification column upper discharge stream 21 containing unreacted isobutyraldehyde, a catalyst, and a catalyst salt and an aldol purification column lower discharge stream containing hydroxypivaldehyde.

Specifically, in the aldol purification column 200, the first reaction product may be distilled to obtain an aldol purification column upper discharge stream 210 containing unreacted isobutyraldehyde, a catalyst, and a catalyst salt and an aldol purification column lower discharge stream 220 containing HPNE and HPA.

According to the present invention, a ratio of a mass flow rate of water contained in the aldol purification column lower discharge stream 220 to a mass flow rate of water contained in the feed stream 1 may be 0.10 to 0.60, and more specifically, 0.15 to 0.50 or 0.20 to 0.30.

Specifically, when the mass flow rate ratio is less than 0.10 wt%, as a content of water contained in the aldol purification column lower discharge stream 220 is reduced, the energy usage in a neopentyl glycol purification column 300 described below may decrease, but the energy usage to maximize the purification efficiency in the aldol purification column 200 may increase excessively. When the mass flow rate ratio exceeds 0.60 wt%, as the content of water contained in the aldol purification column lower discharge stream 220 increases, the energy usage in the aldol purification column 200 may decrease, but the energy usage in the neopentyl glycol purification column 300 described below may increase. Therefore, considering the total energy used in the process, it is preferable to set the mass flow rate ratio to be within an appropriate range. In other words, considering the total energy used in the process, it is preferable to control the mass flow rate ratio to 0.10 to 0.60.

According to an embodiment of the present invention, the content of water contained in the aldol purification column lower discharge stream 220 may be 3 to 20 wt%, and specifically, 5 to 10 wt%. The content of water contained in the aldol purification column lower discharge stream 220 may be controlled by controlling the ratio of the mass flow rate of water contained in the aldol purification column lower discharge stream 220 to the mass flow rate of water contained in the feed stream 1 to 0.10 to 0.60.

Specifically, when the content of water contained in the aldol purification column lower discharge stream 220 is less than 3, a content of water contained in the aldol purification column upper discharge stream 210 may need to be maximized to control the content of water. Thereafter, the aldol purification column upper discharge stream 210 may be fed to the decanter 250, and water contained in the aldol purification column upper discharge stream 210 may be separated into an aqueous phase stream 240 as much as possible. To this end, the amount of energy used in the aldol purification column 200 and the decanter 250 increases excessively, which may not be preferable in terms of economic feasibility.

When the content of water contained in the aldol purification column lower discharge stream 220 exceeds 20, the amount of catalyst salt present in a dissociated state in the water may also increase as much as the content of water contained in the aldol purification column lower discharge stream 220 increases. As such, when water containing a large amount of a catalyst salt is subjected to a hydrogenation reaction together with HPA, a side reaction may occur due to the catalyst salt and a conversion rate to NPG through the hydrogenation reaction may be decreased. Therefore, in accordance with an increase in production of by-products due to the large amount of water and the side reaction in the hydrogenation reactor 30, the energy usage may increase excessively in separating and distilling the by-products in the subsequent process, for example, in the NPG purification column 300.

In order to prepare NPG by reacting the HPA with hydrogen, the HPA needs to be separated from the first reaction product. To this end, an extraction process of extracting HPA from the first reaction product was performed in the related art. However, in the present invention, in separating HPA from the first reaction product, the extraction process is not performed, and HPA is separated through the aldol purification column 200. As described above, as the extraction process is not performed in separating HPA from the first reaction product, there is no need to use an extractant, and the extractant is not contained in a final product, and thus, high-quality NPG may be obtained. There is no need to additionally provide a separate column, for example, an extractant separation column, to finally separate the extractant, and therefore, the process is simplified, which may reduce the overall facility costs and process operating costs.

To this end, an operating temperature of the aldol purification column 200 may be 40°C or higher, 45°C or higher, 50°C or higher, or 55°C or higher, and 85°C or lower, 90°C or lower, 95°C or lower, or 100°C or lower. In addition, an operating pressure of the aldol purification column 200 may be 300 torr or more, 350 torr or more, 400 torr or more, or 450 torr or more, and 600 torr or less, 650 torr or less, 700 torr or less, or 760 torr or less. The aldol purification column 200 is performed within the above temperature and pressure ranges, such that distillation performed in the aldol purification column may proceed smoothly, and separation between TEA, which is a relatively low boiling point material, and HPA, which is a relatively high boiling point material, may be easily performed.

The method for preparing neopentyl glycol according to an embodiment of the present invention may include feeding the aldol purification column upper discharge stream 250 to the decanter 250 and separating an organic phase stream 230 containing the unreacted isobutyraldehyde and the catalyst and an aqueous phase stream 240 containing the catalyst salt.

Specifically, the aldol purification column upper discharge stream 210 may be fed to the decanter 250 provided upstream of the aldol purification column 200, and the aldol purification column upper discharge stream 210 may be separated into the organic phase stream 230 containing the unreacted IBAL and the catalyst and the aqueous phase stream 240 containing the catalyst salt. In this case, water contained in the aqueous phase stream 240 may be derived from the formaldehyde aqueous solution, and the catalyst salt may be present in a dissociated state in the water.

When a multi-stage distillation column was used without an extraction process to separate the first reaction product into the stream containing HPA, the stream containing unreacted IBAL and a catalyst, and the stream containing water, there was a problem in that it was difficult to obtain the stream containing water as an appropriate feed to be fed to the saponification reactor. Specifically, when the multi-stage distillation column was used to separate the first reaction product, the organic matter (for example, unreacted IBAL and catalyst) and water contained in the first reaction product were not separated separately and had to be fed together to the saponification reactor. In this case, in the saponification reactor, excessive by-products due to the organic matter may be produced. Therefore, in order to feed the stream containing water, specifically, the stream containing water from which the catalyst salt is dissociated, to the saponification reactor, as in the present invention, it is preferable that the decanter is provided, the organic phase stream containing organic matter and the aqueous phase stream containing water from which the catalyst salt is dissociated are separated in the decanter, and the aqueous phase stream is fed to the saponification reactor.

More specifically, in the present invention, through the decanter 250, the aldol purification column upper discharge stream 210 may be subjected to layer separation to be separated into each stream suitable for feed to the saponification reactor 20 and a raw material recovery column 500 described below, specifically, an aqueous phase stream and an organic phase stream. In particular, in order to reduce the catalyst salt contained in the aldol purification column upper discharge stream 210 to a catalyst, the catalyst salt needs to be fed to the saponification reactor 20. According to an embodiment of the present invention, the catalyst salt may be fed to the saponification reactor to be reduced to a catalyst, and the catalyst may be recovered and recycled in the aldol reactor 10.

Since the catalyst salt is mainly present in a dissociated state in water, it may be appropriate to separate the aldol purification column upper discharge stream 210 into an aqueous phase and an organic phase through the decanter 250 without providing a distillation column other than the aldol purification column.

Even when the first reaction product may be separated into the stream containing HPA, the stream containing unreacted IBAL and a catalyst, and the stream containing water using the multi-stage distillation column, compared to the present invention in which separation of the first reaction product is performed using the aldol purification column 200 and the decanter 250, energy usage and process facility costs may increase, and it is not preferable in terms of space utilization for the process facilities.

The method for preparing neopentyl glycol according to an embodiment of the present invention may include recovering the unreacted isobutyraldehyde and the catalyst from the organic phase stream 230, and feeding the aqueous phase stream 240 to the saponification reactor to reduce the catalyst salt to a catalyst and recover the catalyst.

Specifically, the organic phase stream 230 may contain the unreacted IBAL and the catalyst, and may be fed to the raw material recovery column 500 described below. The aqueous phase stream 240 may contain the catalyst salt, and may be fed to the saponification reactor 20.

In the saponification reactor 20, the catalyst salt contained in the aqueous phase stream 240 may be reduced to a catalyst. The catalyst salt may be formed by side reactions caused by the aldol condensation reaction as described above.

The saponification reaction may be performed by the reaction of the catalyst salt with an inorganic strong base such as sodium hydroxide (NaOH) that is added separately, thereby efficiently reusing the reduced catalyst. For example, when TEA is used as a catalyst for the aldol condensation reaction, a TEA salt may be reduced to TEA according to the following Reaction Scheme 1.

[Reaction Scheme 1] TEA-Salt + NaOH → TEA + Na-Salt

A temperature of the saponification reaction in the saponification reactor 20 may be 50°C or higher, 55°C or higher, or 60°C or higher, and 90°C or lower, 95°C or lower, or 100°C or lower. When the saponification reaction temperature is lower than 50°C, the saponification reaction may not proceed smoothly due to a low temperature, and thus, a conversion rate to the catalyst may be decreased. When the saponification reaction temperature exceeds 100°C, excessive by-products may be produced during the saponification reaction.

When the catalyst salt is not reduced to a catalyst through the saponification reaction and is fed in large quantities to the subsequent process, for example, the hydrogenation reactor 30 described below, in the state of a catalyst salt, the hydrogenation reaction performed in the hydrogenation reactor 30 may be adversely affected. Therefore, the catalyst salt needs to be removed by reduction to a catalyst in advance, and the reduced catalyst needs to be recovered. Specifically, under conditions for the hydrogenation reaction in the hydrogenation reactor 30, the catalyst salt may interfere with the role of a hydrogenation reaction catalyst required for the hydrogenation reaction and may cause various side reactions, which may ultimately decrease the conversion rate of the hydrogenation reaction.

Therefore, the catalyst salt is reduced to a catalyst in the saponification reactor 20 and the catalyst is recovered in the raw material recovery column 500 described below, such that the introduction of the catalyst salt into the hydrogenation reactor 30 may be prevented, thereby minimizing the various side effects described above.

In addition, the catalyst salt is reduced to a catalyst in the saponification reactor 20, such that the catalyst may be efficiently reused, thereby securing cost competitiveness of the process. Furthermore, it is possible to solve the environmental pollution problem that may occur when the catalyst salt is disposed of without being reduced.

A saponification reactor discharge stream 21 containing the reduced catalyst, for example, TEA, may then be fed to the raw material recovery column 500.

According to the present invention, the recovering of the unreacted isobutyraldehyde and the catalyst from the organic phase stream 230 and feeding of the aqueous phase stream 240 to the saponification reactor 20 to reduce the catalyst salt to a catalyst and recover the catalyst may be performed through the raw material recovery column 500.

The present invention may further include feeding the organic phase stream 230 and the saponification reactor discharge stream 21 containing the reduced catalyst to the raw material recovery column 500, and feeding a raw material recovery column upper discharge stream 510 containing unreacted isobutyraldehyde and the catalyst to the aldol reactor 10.

According to an embodiment of the present invention, the organic phase stream 230, the saponification reactor discharge stream 21, and a stream 310 containing the catalyst separated from the neopentyl glycol purification column may be distilled in the raw material recovery column 500, and the raw material recovery column upper discharge stream 510 containing unreacted IBAL and the catalyst may be fed to the aldol reactor 10.

Specifically, the feed (the organic phase stream 230, the saponification reactor discharge stream 21, and the stream 310 containing the catalyst separated from the neopentyl glycol purification column described below) fed to the raw material recovery column 500 is distilled to obtain a raw material recovery column lower discharge stream 520 containing impurities (i-BuOH, MeOH, and unidentified by-products) and water and a raw material recovery column upper discharge stream 510 containing unreacted IBAL and the catalyst. The raw material recovery column upper discharge stream 510 may be fed to the aldol reactor 10.

The unreacted IBAL and the catalyst contained in the raw material recovery column upper discharge stream 510 are separated from the impurities and water in the raw material recovery column 500, and may thus be suitable for reuse as raw materials for the aldol condensation reaction in the aldol reactor 10. In addition, since the catalyst and IBAL used in the aldol reactor 10 are reused, the amount of newly added raw materials may be reduced, and the production costs used during the process may be reduced.

To this end, an operating temperature of the raw material recovery column 500 may be 50°C to 80°C, 55°C to 75°C, or 60°C to 70°C. In addition, an operating pressure of the raw material recovery column 500 may be 400 torr to 760 torr, and specifically, 430 torr to 700 torr or 470 torr to 650 torr. When the raw material recovery column 500 is operated within the above temperature and pressure ranges, the unreacted IBAL and the catalyst contained in the feed fed to the raw material recovery column 500 may be separated from the water and by-products and then recovered, and thus, the economic feasibility in terms of raw material utilization may be improved.

The saponification reactor discharge stream 21 and the organic phase stream 230 may be fed to the raw material recovery column 500, and distillation of unreacted IBAL and TEA may be performed by one recovery column instead of applying separate recovery columns for the respective streams as in the related art. This is because the contents of TEA contained in the saponification reactor discharge stream 21 and the organic phase stream 230 are similar, such that TEA and IBAL having a boiling point lower than that of TEA may be distilled simultaneously by the raw material distillation column 500.

According to an embodiment of the present invention, a ratio of a content of the catalyst in the saponification reactor discharge stream 21 to a content of the catalyst in the organic phase stream 230 may be 0.60 to 1.20, and specifically, 0.65 to 1.15 or 0.70 to 1.10. By feeding streams having similar catalyst compositions in the streams to the raw material recovery column 500, distillation and separation performed in separate recovery columns for the respective streams may be performed in one recovery column.

The method for preparing neopentyl glycol according to an embodiment of the present invention may include feeding the aldol purification column lower discharge stream 220 to the hydrogenation reactor 30 and subjecting the aldol purification column lower discharge stream 220 to a hydrogenation reaction to obtain a second reaction product containing neopentyl glycol.

Specifically, the aldol purification column lower discharge stream 220 described above may be fed to the hydrogenation reactor 30, and the hydrogenation reaction may be performed in the hydrogenation reactor 30. The hydrogenation reaction may be performed by the reaction of HPA with additionally added hydrogen in the presence of a hydrogenation reaction catalyst. By such a hydrogenation reaction, a second reaction product containing NPG, which is a product, may be obtained.

According to the present invention, the second reaction product may further contain a catalyst, a catalyst salt, neopentyl glycol, and HPNE. The HPNE may be produced as a by-product of the aldol condensation reaction in the aldol reactor 10, and the catalyst and catalyst salt may be small amounts of a catalyst and a catalyst salt that are not separated in the aldol purification column 200. In this case, the catalyst salt may be present in a dissociated state in water, and the water may not be separated into the aqueous phase stream 240 in the aldol purification column 200.

As described above, when a large amount of a catalyst salt, for example, a TEA salt, formed through the aldol condensation reaction is introduced into the hydrogenation reactor 30, various side reactions may occur, thereby decreasing the conversion rate of the hydrogenation reaction. Therefore, the catalyst salt is reduced to a catalyst and recovered by the saponification reactor 20 of the present invention, and a content of water in the aldol purification column lower discharge stream 220 is controlled to (a) to (b) wt% by controlling a ratio of a mass flow rate of water contained in the aldol purification column lower discharge stream 220 to a mass flow rate of water contained in the first reaction product, such that the amount of catalyst salt introduced into the hydrogenation reactor 30 may be reduced, thereby improving the conversion rate of the hydrogenation reaction. That is, as the introduction amount of TEA salt, which causes poisoning of the hydrogenation reaction catalyst, that is, catalyst poison, is reduced, the hydrogenation reaction may be activated.

The hydrogenation reaction may be performed at a hydrogen pressure of 100 to 1,500 psig (pounds per square inch gauge pressure) and a reaction temperature of 100°C to 200°C. In addition, as the hydrogenation reaction catalyst, a copper catalyst or a nickel catalyst may be used. As an example, the copper catalyst may be a CuO/BaO/SiO catalyst, and the CuO/BaO/SiO catalyst may be a catalyst of (CuO)x(BaO)y(SiO)z (x, y, and z are wt%, and x:y:z = 10 to 50:0 to 10:40 to 90, 10 to 50:1 to 10:40 to 89, or 29 to 50:1 to 10:40 to 70). The sum of x and y is preferably 20 to 50 (wt%) or 30 to 50 (wt%), based on the total sum of x, y, and z (100 wt%), and within this range, the performance of the hydrogenation reaction catalyst is excellent and the lifespan is long. The amount of the nickel catalyst may be 2 to 10 wt% with respect to the weight of HPA.

The method for preparing neopentyl glycol according to an embodiment of the present invention may include obtaining the neopentyl glycol from the second reaction product.

Specifically, the obtaining of the neopentyl glycol from the second reaction product may include feeding the second reaction product to the neopentyl glycol purification column 300 and distilling the second reaction product to separate a stream 330 containing neopentyl glycol, a stream 310 containing a catalyst, a stream 340 containing a catalyst salt, and a stream 320 containing hydroxypivalic acid-neopentylglycol ester; and obtaining the neopentyl glycol from the separated stream 330 containing the neopentyl glycol, feeding the separated stream 310 containing the catalyst to the raw material recovery column 500, and feeding the separated stream 340 containing the catalyst salt to the saponification reactor 20.

More specifically, the second reaction product may be fed as a hydrogenation reactor discharge stream 21 to the NPG purification column 300, and distillation may be performed. In this case, the NPG purification column 300 may be one or two or more purification columns.

First, when the NPG purification column 300 is one purification column, a catalyst and a catalyst salt, HPNE, and NPG may be separated from an upper portion, a lower portion, and a side portion of the one purification column, respectively. In this case, the NPG may be obtained from a side portion at a height of 40% to 80% from the uppermost portion of the NPG purification column to a lower position. In addition, the NPG purification column may be, for example, one dividing wall distillation column. When the NPG purification column 300 is two or more purification columns, the operation may be performed through one or more NPG purification columns for separating a stream containing a catalyst to an upper portion and one or more NPG purification columns for separating a stream containing a catalyst salt to an upper portion. A stream containing HPNE or NPG may be separated and discharged from lower portions of the two or more NPG purification columns. That is, the second reaction product may be separated into a catalyst, a catalyst salt, HPNE, and NPG by the one or two or more NPG purification columns.

NPG may be finally obtained from the separated stream 330 containing the NPG. As described above, as an extractant is not used in the process, the NPG may be obtained with a high purity, and production of by-products that may be newly produced due to the use of the extractant may be prevented. Furthermore, when an extractant is used as in the related art, another distillation column should be additionally provided to separate the extractant from NPG, recover the extractant separately, and then circulate the extractant to the column where the extraction process is performed. However, in a case where an extractant is not used as in the present invention, there is no need to provide such a distillation column. That is, since there is no need to additionally provide at least two columns that should be provided when using an extractant, the process design is simpler than in the related art, and thus, the facility space may be minimized and the energy usage involved in the process may be reduced.

The separated stream 310 containing the catalyst may be fed to the raw material recovery column 500, and the separated stream 340 containing the catalyst salt may be fed to the saponification reactor 20. In this case, the separated catalyst salt may be present in the form of ions in water.

As such, the catalyst and the catalyst salt separated from the NPG purification column 300 are fed to the raw material recovery column 500 and the saponification reactor 30, respectively, such that the catalyst and the catalyst salt that are present in the system may be recovered. Furthermore, the separated catalyst salt fed to the saponification reactor 30 may be reduced to a catalyst and reused. In the related art, the stream containing the catalyst salt was treated as wastewater. However, in the present invention, as described above, the stream containing the catalyst may be fed to the saponification reactor 20 and the reduced catalyst may be recovered. Through this, a recovery rate of the catalyst used in the aldol condensation reaction in the aldol reactor 10 may be further increased.

An operating temperature of the NPG purification column 300 may be 60°C or higher, 80°C or higher, 100°C or higher, 120°C or higher, or 140°C or higher, and 180°C or lower, 185°C or lower, 190°C or lower, 195°C or lower, or 200°C or lower. In addition, an operating pressure of the NPG purification column 300 may be 40 torr or more, 90 torr or more, 120 torr or more, or 140 torr or more, and 300 torr or less, 400 torr or less, 500 torr or less, or 600 torr or less. When the NPG purification column 300 is operated within the above temperature and pressure ranges, the catalyst salt, the catalyst, NPG, and HPNE may be easily separated. Therefore, a content of by-products present in the stream 330 containing NPG to be obtained in the present invention may be reduced, and thus, high-purity NPG may be obtained.

As the HPNE produced by side reactions of the aldol condensation reaction is partially reduced to NPG in the hydrogenation reactor 30 and the remainder remains as HPNE, the HPNE may be introduced into the NPG purification column 300, which may decrease the NPG yield. Therefore, the HPNE is fed to an HPNE purification column 400 described below, such that NPG may be additionally obtained, and the HPNE, which is a high-value-added product in itself, may be recovered separately and utilized. That is, the HPNE may be commercialized separately from other heavy by-products, for example, trimethylpentanediol (2,2,4-trimethyl-1,3-pentanediol (TMPD)), which is a by-product of the hydrogenation reaction.

The method for preparing neopentyl glycol according to an embodiment of the present invention may further include feeding the stream 320 containing HPNE separated from the NPG purification column to the HPNE purification column 400 and separating HPNE and NPG and obtaining the HPNE; and feeding the NPG to the NPG purification column.

Specifically, the stream 320 containing HPNE separated from the NPG purification column may further contain not only HPNE but also a trace of NPG that is not separated in the NPG purification column 300. Therefore, the stream 320 containing HPNE separated from the NPG purification column may be fed to the HPNE purification column 400, and distillation may be performed, thereby separating an HPNE purification column upper discharge stream 410 containing the trace of NPG, an HPNE purification column side discharge stream 430 containing HPNE, and an HPNE purification column lower discharge stream 420 containing high boiling point by-products.

HPNE may be obtained from the HPNE purification column side discharge stream 430. Specifically, the HPNE may be obtained from a side portion at a height of 50% to 80% from the uppermost portion of the HPNE purification column to a lower position. When HPNE is obtained at a height within the above range, contents of other materials, for example, NPG and high boiling point by-products, in the HPNE purification column side discharge stream 430 may be minimized, and HPNE may be obtained to the maximum.

As described above, the obtained HPNE may be recovered and utilized in various ways. For example, the obtained HPNE may be used as a main raw material for the synthesis and coating of polyester as a high-value-added product. As such, since the HPNE may be used as a raw material in other processes, the economic feasibility in terms of raw material utilization may be improved by the HPNE purification column 400 according to the present invention.

The HPNE purification column upper discharge stream 410 may be fed to the NPG purification column 400. Accordingly, NPG that is discharged without being recovered in the NPG purification column 300 may be recovered from the upper portion of the HPNE purification column 400 (410), and thus, the recovery rate of NPG may be further increased through the HPNE purification column 400.

An operating temperature of the HPNE purification column 400 may be 100°C or higher, 110°C or higher, or 120°C or higher, and 230°C or lower, 240°C or lower, or 250°C or lower. In addition, an operating pressure of the HPNE purification column 400 may be 40 torr or more, 45 torr or more, or 50 torr or more, and 150 torr or less, 160 torr or less, or 170 torr or less. When the HPNE purification column 400 is operated within the above temperature and pressure ranges, separation of HPNE and NPG is smoothly performed, such that NPG to be obtained in the present invention may be additionally obtained, and HPNE may also be obtained as a high-value-added product.

Hereinafter, the present invention will be described in more detail with reference to examples. However, the following examples are provided for illustrating the present invention. It is apparent to those skilled in the art that various modifications and alterations may be made without departing from the scope and spirit of the present invention, and the scope of the present invention is not limited thereto.

### Examples

### Example 1

According to the process flow illustrated in FIG. 1, a neopentyl glycol (NPG) preparation process was simulated using Aspen Plus simulator available from Aspen Technology, Inc.

Specifically, a feed stream 1 containing formaldehyde (FA) and isobutyraldehyde (IBAL) was fed to an aldol reactor 10 and an aldol condensation reaction was performed in the presence of a catalyst (TEA) to obtain a first reaction product containing hydroxypivaldehyde (HPA). At this time, the formaldehyde was a formaldehyde aqueous solution, and the aldol condensation reaction was performed at a temperature of 85°C.

The first reaction product was fed to an aldol purification column 200, and an aldol purification column upper discharge stream 210 containing unreacted IBAL, a catalyst, and a catalyst salt (TEA salt) and an aldol purification column lower discharge stream 220 containing HPA were obtained. At this time, the aldol purification column 200 was operated at an operating temperature of 87°C and an operating pressure of 207 torr.

The ratio of the mass flow rate of water contained in the aldol purification column lower discharge stream 220 to the mass flow rate of water contained in the feed stream 1 was 0.278. The content of water contained in the aldol purification column lower discharge stream 220 was 7.5 wt%.

The aldol purification column upper discharge stream 210 was fed to a decanter 250, and an organic phase stream 230 containing the unreacted IBAL and the catalyst and an aqueous phase stream 240 containing the catalyst salt were separated. The organic phase stream 230 was fed to a raw material recovery column 500, and the aqueous phase stream 240 was fed to a saponification reactor 20.

In the saponification reactor 20, the catalyst salt contained in the aqueous phase stream 240 was reduced to a catalyst by a reaction with sodium hydroxide (NaOH), and a saponification reactor discharge stream 21 containing the catalyst was fed to the raw material recovery column 500. At this time, the saponification reaction performed in the saponification reactor 20 was performed at a temperature of 83°C.

The ratio of the content of the catalyst in the saponification reactor discharge stream 21 to the content of the catalyst in the organic phase stream 230 described above was 0.9.

In the raw material recovery column 500, the organic phase stream 230, the saponification reactor discharge stream 21, and a stream 310 containing a catalyst separated from an NPG purification column 300 described below were distilled to obtain a raw material recovery column upper discharge stream 510 containing unreacted IBAL and the catalyst, and a raw material recovery column lower discharge stream 520 containing water and impurities. The raw material recovery column upper discharge stream 510 was circulated to the aldol reactor 10. At this time, the raw material recovery column 500 was operated at an operating temperature of 68.5°C and an operating pressure of 510 torr.

The aldol purification column lower discharge stream 220 was fed to a hydrogenation reactor 30 and subjected to a hydrogenation reaction to obtain a second reaction product containing NPG. At this time, the hydrogenation reaction was performed at a temperature of 160°C. A catalyst, a catalyst salt, NPG, and hydroxypivalic acid-neopentylglycol ester (HPNE) were contained in the second reaction product.

The second reaction product 31 was fed to the NPG purification column 300, and distillation was performed to separate a stream 310 containing a catalyst, a stream 320 containing HPNE, a stream 330 containing NPG, and a stream 340 containing a catalyst salt. At this time, the NPG purification column was operated at an operating pressure of 154 torr and an operating temperature of 168°C.

NPG was obtained from the separated stream 330 containing the NPG. The separated stream 310 containing the catalyst was fed to the raw material recovery column 500, the separated stream 340 containing the catalyst salt was fed to the saponification reactor 20, and the separated stream 320 containing the HPNE was fed to an HPNE purification column 400.

In the HPNE purification column 400, the separated stream 320 containing the HPNE was distilled to separate an HPNE purification column side discharge stream 430 containing HPNE, an HPNE purification column upper discharge stream 410 containing a trace of NPG, and an HPNE purification column lower discharge stream 420 containing high boiling point by-products. HPNE was obtained from the HPNE purification column side discharge stream 430, and the HPNE purification column upper discharge stream 410 was circulated to the NPG purification column 300. At this time, the HPNE was obtained from a side portion at a height of 70% from the uppermost portion of the HPNE purification column 400 to a lower position.

### Comparative Examples

### Comparative Example 1

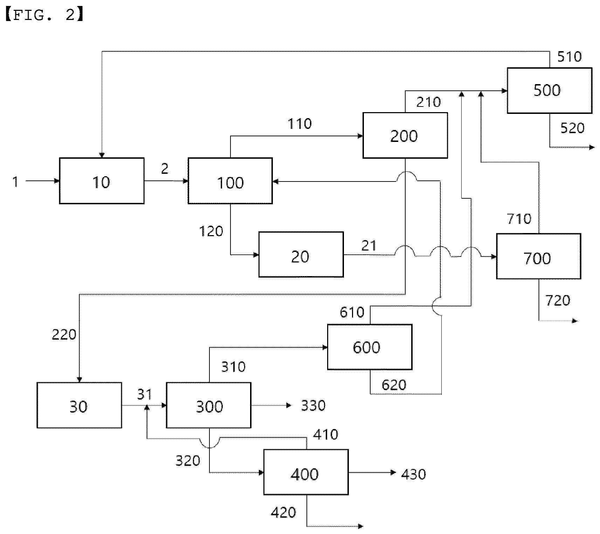

According to the process flow illustrated in FIG. 2, a neopentyl glycol (NPG) preparation process was simulated using Aspen Plus simulator available from Aspen Technology, Inc.

Specifically, a feed stream 1 containing FA and IBAL was fed to an aldol reactor 10 and an aldol condensation reaction was performed in the presence of a catalyst (TEA) to obtain a first reaction product containing HPA. At this time, the aldol condensation reaction was performed at a temperature of 85°C.

The first reaction product was fed to an aldol extraction column 100 and brought into contact with an extractant (2-ethylhexanol (2-EH)) to obtain an extract containing HPA and a raffinate containing a catalyst salt. At this time, the aldol extraction column 100 was operated at an operating temperature of 65°C and an operating pressure of 760 torr.

The raffinate was fed as a raffinate stream 120 to a saponification reactor 20, the catalyst salt was reacted with sodium hydroxide (NaOH) to be reduced to a catalyst, and a saponification reactor discharge stream 21 containing the reduced catalyst was fed to a catalyst recovery column 700. At this time, the saponification reaction performed in the saponification reactor was performed at a temperature of 83°C.

The saponification reactor discharge stream 21 was distilled in the catalyst recovery column 700 to remove water and by-products (720), thereby obtaining a stream containing a catalyst, from which water and by-products were removed. The stream containing the catalyst, from which water and by-products were removed, was fed as a catalyst recovery column upper discharge stream 710 to a raw material recovery column 500.

An extract stream 110 containing the extract described above was fed to an aldol purification column 200, and distillation was performed to obtain an aldol purification column lower discharge stream 220 containing HPA and an aldol purification column upper discharge stream 210 containing unreacted IBAL and a catalyst. The ratio of the mass flow rate of water contained in the aldol purification column lower discharge stream 220 to the mass flow rate of water contained in the feed stream 1 was 0.278. At this time, the aldol purification column 200 was operated at an operating pressure of 207 torr and an operating temperature of 87°C. The aldol purification column upper discharge stream 210 was fed to the raw material recovery column 500, and the aldol purification column lower discharge stream 220 was fed to a hydrogenation reactor 30.

In the raw material recovery column 500, the aldol purification column upper discharge stream 210, the catalyst recovery column upper discharge stream 710, and an extractant recovery column upper discharge stream 610 described below were distilled to remove impurities (520), thereby obtaining a stream containing unreacted IBAL and a catalyst, from which the impurities were removed. The stream containing the unreacted isobutyraldehyde and the catalyst, from which the impurities were removed, was fed as a raw material recovery column upper discharge stream 510 to the aldol reactor 10.

The aldol purification column lower discharge stream 220 was fed to the hydrogenation reactor 30 and subjected to a hydrogenation reaction to obtain a second reaction product containing NPG. At this time, the hydrogenation reaction was performed at a temperature of 160°C.

The second reaction product was fed to a neopentyl glycol purification column 300, and a stream 310 containing a catalyst and an extractant, a stream 330 containing NPG, and a stream 320 containing HPNE were separated. At this time, the neopentyl glycol purification column was operated at an operating pressure of 154 torr and an operating temperature of 168°C. NPG was obtained from the stream 330 containing NPG, the stream 310 containing the catalyst and the extractant was fed to an extractant recovery column 600, and the stream 320 containing HPNE was fed to an HPNE purification column 400 described below.

In the extractant recovery column 500, the stream 310 containing the catalyst and the extractant was distilled, an extractant recovery column upper discharge stream 510 containing a catalyst was fed to the aldol purification column 200, and an extractant recovery column lower discharge stream 520 containing an extractant was fed to the aldol extraction column 100.

In the HPNE purification column 400, the stream 320 containing HPNE was distilled to obtain an HPNE purification column upper discharge stream 410 containing NPG, an HPNE purification column side discharge stream 430 containing HPNE, and an HPNE purification column lower discharge stream 420 containing high boiling point by-products. The HPNE purification column upper discharge stream 410 was fed to the NPG purification column 300, and HPNE was obtained from the HPNE purification column side discharge stream 430.

### Comparative Example 2

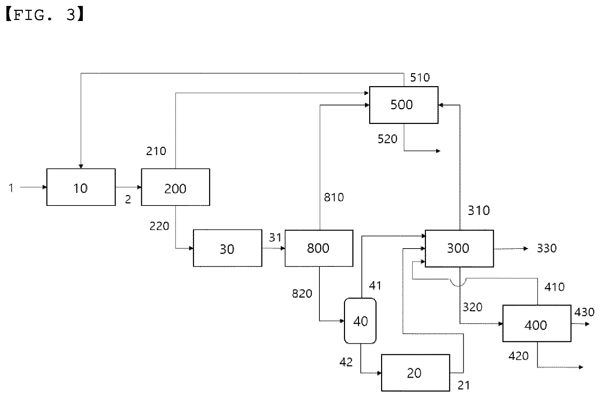

According to the process flow illustrated in FIG. 3, a neopentyl glycol (NPG) preparation process was simulated using Aspen Plus simulator available from Aspen Technology, Inc.

In Comparative Example 2, a first reaction product 2 obtained by the same process as that of Example 1 was fed to an aldol purification column 200, and an aldol purification column upper discharge stream 210 containing unreacted IBAL and a catalyst and an aldol purification column lower discharge stream 220 containing a catalyst salt (TEA salt) and HPA were obtained. The aldol purification column upper discharge stream 210 was fed to a raw material recovery column 500.

The aldol purification column lower discharge stream 220 was fed to a hydrogenation reactor 30 and subjected to a hydrogenation reaction to obtain a second reaction product containing NPG. A catalyst, a catalyst salt, NPG, and hydroxypivalic acid-neopentylglycol ester (HPNE) were contained in the second reaction product.

The second reaction product 31 was fed to a low boiling point by-product removal column 800, and a low boiling point by-product removal column upper discharge stream 810 containing a catalyst and a low boiling point by-product removal column lower discharge stream 820 containing a catalyst salt, NPG, and HPNE were obtained. The low boiling point by-product removal column upper discharge stream 810 was fed to the raw material recovery column 500, and the low boiling point by-product removal column lower discharge stream 820 was fed to a flash drum 40.

In the raw material recovery column 500, the aldol purification column upper discharge stream 210, the low boiling point by-product removal column upper discharge stream 810, and a stream 310 containing a catalyst separated from an NPG purification column 300 described below were distilled to obtain a raw material recovery column upper discharge stream 510 containing unreacted IBAL and the catalyst, and a raw material recovery column lower discharge stream 520 containing water and impurities. The raw material recovery column upper discharge stream 510 was circulated to the aldol reactor 10.

In the flash drum 40, the low boiling point by-product removal column lower discharge stream 820 was distilled to separate a vapor phase stream 41 containing NPG and HPNE and an aqueous phase stream 42 containing the catalyst salt. The vapor phase stream 41 was fed to the NPG purification column 300.

The aqueous phase stream 42 was fed to a saponification reactor 20 to reduce the catalyst salt to a catalyst, and a saponification reactor discharge stream 21 containing the reduced catalyst was fed to the NPG purification column 300.

In the NPG purification column 300, the vapor phase stream 41 and the saponification reactor discharge stream 21 were distilled to separate a stream 310 containing a catalyst, a stream 320 containing HPNE, a stream 330 containing NPG, and a stream 340 containing a catalyst salt.

NPG was obtained from the separated stream 330 containing the NPG. The separated stream 310 containing the catalyst was fed to the raw material recovery column 500, and the separated stream 320 containing the HPNE was fed to an HPNE purification column 400.

In the HPNE purification column 400, the separated stream 320 containing the HPNE was distilled to separate an HPNE purification column side discharge stream 430 containing HPNE, an HPNE purification column upper discharge stream 410 containing a trace of NPG, and an HPNE purification column lower discharge stream 420 containing high boiling point by-products. HPNE was obtained from the HPNE purification column side discharge stream 430, and the HPNE purification column upper discharge stream 410 was circulated to the NPG purification column 300.

Table 1 shows the mass flow rate ratios, FA flow rate ratios (FA/product), IBAL flow rate ratios (IBAL/product), and steam flow rate ratios (steam/product) in examples and comparative examples.

The mass flow rate ratio represents the ratio of the mass flow rate of water contained in the aldol purification column lower discharge stream to the mass flow rate of water contained in the feed stream.

The FA flow rate ratio (FA/product) is the ratio of the mass flow rate of FA contained in the feed stream to the mass flow rate of NPG contained in the stream containing NPG in the NPG purification column, and is represented by converting the FA flow rate ratio of Comparative Example 1 to 100%.

The IBAL flow rate ratio (IBAL/product) is the ratio of the mass flow rate of IBAL contained in the feed stream to the mass flow rate of NPG contained in the stream containing NPG in the NPG purification column, and is represented by converting the IBAL flow rate ratio of Comparative Example 1 to 100%.

The steam flow rate ratio (steam/product) is the ratio of the total mass flow rate of the steam used in the process to the mass flow rate (ton/hr) of NPG contained in the stream containing NPG in the NPG purification column, and is represented by converting the steam flow rate ratio of Comparative Example 1 to 100%.

**[Table 1]**

| | Mass flow rate ratio | FA flow rate ratio (FA/product) | IBAL flow rate ratio (IBAL/product) | Steam flow rate ratio (Steam/product) | Use of extractant | Whether or not extractant is contained in product (NPG) |
|---|---|---|---|---|---|---|
| Example 1 | 0.278 | 100% | 100% | 97% | x | x |
| Comparative Example 1 | 0.278 | 100% | 100% | 100% | ○ | ○ |
| Comparative Example 2 | 0.5 | 104% | 103% | 166% | x | x |

Referring to Table 1, it was confirmed that the FA flow rate ratio and the IBAL flow rate ratio in Example 1 were lower than those in Comparative Example 2. This may mean that the aldol condensation reaction of FA and IBAL in Example 1 is performed more smoothly than in Comparative Example 3. In addition, in Example 1, it was confirmed that the steam flow rate ratio was the lowest, and through this, it was confirmed that the total energy used in the process was reduced.

In Comparative Example 1 in which an extraction process was performed using an extractant, it was confirmed that the extractant (2-EH) was contained in the finally obtained NPG product, and the steam flow rate ratio was increased compared to Example 1.

In Comparative Example 2 in which a saponification reaction was performed after a hydrogenation reaction, the FA flow rate ratio and the IBAL flow rate ratio were the highest, and thus, it was confirmed that the amounts of FA and IBAL fed to the aldol reactor were the highest compared to the amount of NPG finally obtained. That is, this may mean that the reactivity of FA and IBAL is the lowest in the aldol reactor. In addition, it was confirmed that the steam flow rate ratio was also the highest among the examples and the comparative examples.

### [Detailed Description of Main Elements]

10: Aldol reactor 20: Saponification reactor
30: Hydrogenation reactor
200: Aldol purification column 250: Decanter
300: NPG purification column 400: HPNE purification column
500: Raw material recovery column 600: Extractant recovery column
700: Catalyst recovery column
800: Low boiling point by-product removal column 40: Flash drum

## Claims

1. A method for preparing neopentyl glycol, the method comprising:
feeding a feed stream containing formaldehyde and isobutyraldehyde to an aldol reactor and subjecting the feed stream to an aldol condensation reaction in the presence of a catalyst to obtain a first reaction product containing hydroxypivaldehyde (HPA);
feeding the first reaction product to an aldol purification column and obtaining an aldol purification column upper discharge stream containing unreacted isobutyraldehyde, the catalyst, and a catalyst salt and an aldol purification column lower discharge stream containing hydroxypivaldehyde;
feeding the aldol purification column upper discharge stream to a decanter and separating an organic phase stream containing the unreacted isobutyraldehyde and the catalyst and an aqueous phase stream containing the catalyst salt;
recovering the unreacted isobutyraldehyde and the catalyst from the organic phase stream, and feeding the aqueous phase stream to a saponification reactor to reduce the catalyst salt to a catalyst and recovering the catalyst;
feeding the aldol purification column lower discharge stream to a hydrogenation reactor and subjecting the aldol purification column lower discharge stream to a hydrogenation reaction to obtain a second reaction product containing neopentyl glycol; and
obtaining the neopentyl glycol from the second reaction product.

2. The method of claim 1, wherein the catalyst includes one or more of trialkylamine, trimethylamine, tripropylamine, triisopropylamine, tributylamine, and triethylamine (TEA).

3. The method of claim 1, wherein a ratio of a mass flow rate of water contained in the aldol purification column lower discharge stream to a mass flow rate of water contained in the feed stream is 0.10 to 0.60.

4. The method of claim 1, wherein a content of water contained in the aldol purification column lower discharge stream is 3 to 20 wt%.

5. The method of claim 1, wherein an operating temperature of the aldol purification column is 40 to 100°C, and
an operating pressure of the aldol purification column is 300 to 760 torr.

6. The method of claim 1, wherein the recovering of the unreacted isobutyraldehyde and the catalyst from the organic phase stream and feeding of the aqueous phase stream to the saponification reactor to reduce the catalyst salt to a catalyst and recovering the catalyst is performed through a raw material recovery column, and
the method further comprises feeding the organic phase stream and a saponification reactor discharge stream containing the reduced catalyst to the raw material recovery column, and feeding a raw material recovery column upper discharge stream containing unreacted isobutyraldehyde and the catalyst to the aldol reactor.

7. The method of claim 1, wherein a ratio of a content of the catalyst in the saponification reactor discharge stream to a content of the catalyst in the organic phase stream is 0.60 to 1.20.

8. The method of claim 1, wherein the second reaction product further contains a catalyst, a catalyst salt, and hydroxypivalic acid-neopentylglycol ester (HPNE).

9. The method of claim 7 or 8, wherein the obtaining of the neopentyl glycol from the second reaction product includes:
feeding the second reaction product to a neopentyl glycol purification column and distilling the second reaction product to separate a stream containing neopentyl glycol, a stream containing a catalyst, a stream containing a catalyst salt, and a stream containing hydroxypivalic acid-neopentylglycol ester; and
obtaining the neopentyl glycol from the separated stream containing the neopentyl glycol, feeding the separated stream containing the catalyst to the raw material recovery column, and feeding the separated stream containing the catalyst salt to the saponification reactor.

10. The method of claim 9, further comprising:
feeding the stream containing the hydroxypivalic acid-neopentylglycol ester separated in the neopentyl glycol purification column to a hydroxypivalic acid-neopentylglycol ester purification column, separating hydroxypivalic acid-neopentylglycol ester and neopentyl glycol, and obtaining the hydroxypivalic acid-neopentylglycol ester; and
feeding the neopentyl glycol to the neopentyl glycol purification column.
